# EUROPEAN PATENT SPECIFICATION

(11) **EP 1 100 868 B1**
(45) Date of publication and mention of the grant of the patent: **06.12.2006**
(21) Application number: 99935013.5
(22) Date of filing: 26.07.1999
(51) Int. Cl.: C12N 1/20, C12N 1/04, C12R 1/46

(54) **PROCESS FOR PREPARING STARTER CULTURES OF LACTIC ACID BACTERIA**
VERFAHREN ZUR HERSTELLUNG VON MILCHSÄUREBAKTERIELLEN STARTERKULTUREN
PROCEDE RELATIF A L'ELABORATION DE CULTURES STARTER A BASE DE BACTERIES LACTIQUES

(30) Priority: 24.07.1998 FR 9809463
(43) Date of publication of application: 23.05.2001
(73) Proprietor: INSTITUT NATIONAL DE LA RECHERCHE AGRONOMIQUE (INRA), 75338 Paris Cédex 07 (FR)
(72) Inventor: DUWAT, Patrick, 92120 Montrouge (FR); SOURICE, Sophie, 44000 Nantes (FR); GRUSS, Alexandra, 91400 Orsay (FR)
(74) Representative: Vialle-Presles, Marie José
(86) International application number: PCT/IB1999/001430
(87) International publication number: WO 2000/005342

(56) References cited:
- EP-A- 0 430 406
- US-A- 2 966 445
- DATABASE WPI Section Ch, Week 9212 Derwent Publications Ltd., London, GB; Class B04, AN 92-092889 XP002105963 & JP 04 036180 A (MEIJI MILK PROD CO LTD), 6 February 1992 (1992-02-06) cited in the application
- DATABASE FSTA [Online] INTERNATIONAL FOOD INFORMATION SERVICE (IFIS), FRANFURT/MAIN, DE LINDGREN B ET AL: "Milk with reduced lactose content for preparation of lactic acid starter culture concentrates." XP002098993 & XIX INTERNATIONAL DAIRY CONGRESS, vol. 1E, 1974, Swedish Dairies' Assoc., Malmö, Sweden

## Description

The present invention relates to a novel process for preparing lactic acid bacterial starter cultures that exhibit preservation and acidification properties superior to those of conventionally used starter cultures.

The preparation of fermented products begins with the inoculation of a food substance with a starter culture that consists of one or more bacterial strains having the desired characteristics for producing the final product.

Ready-to-use starter cultures are marketed in the form of bacterial preparations that are generally frozen or lyophilized. In order to ensure that the fermentation starts off well, the bacteria comprising the starter culture should first be cultured, harvested, packaged and stored under conditions that are optimal for their growth and also for their survival. Furthermore, in order to obtain a final product of constant quality, the conditions for preparing the starter culture should be reproducible.

However, different stress conditions that may occur during the different steps of preparing starter cultures can result in altered growth and/or survival of the bacteria.

First of all, when lactic acid bacteria are cultured, the medium becomes acidified as a natural consequence of bacterial growth. This acidification arrests cell division when the pH of the medium reaches a value of around 4.5, and also decreases cell viability.

In addition, aside from lactic acid, lactic acid bacteria also produce various other antibacterial substance during growth, such as nisin, bacteriocins, various organic acids and diacetyl. Since these substances are more active on certain contaminating bacteria that may be present in the culture, than on the lactic acid bacteria themselves, their production in the medium initially favours growth of the latter. However, accumulation of these substances during the culture can also become detrimental to the survival of the lactic acid bacteria.

KANEKO et al. [Appl. Environ. Microbiol., 56:9, 2644-2649 (1990)], describe the culturing of a strain of lactic acid bacteria which possesses a high NADH oxidase activity and a high diacetyl synthase activity (strain 3022 of *L. lactis* subsp. *lactis* biovar *diacetylactis*), under aerobic conditions and in the presence of hemin and/or Cu²⁺. They observe a substantial increase in the production of diacetyl and acetoin. In the course of the culture, they also observe a rise in pH, following an initial drop, the overall result being a decline in the acidification of the culture medium and an increase in bacterial growth, and hence a more substantial bacterial mass (measured by turbidimetry). They show that these effects are mainly due to activation of the diacetyl synthase by the hemin and/or the Cu²⁺, and from an increase of NADH oxidase activity. These conditions promote transformation of the pyruvate (resulting from glycolysis) into diacetyl, to the detriment of lactate formation; furthermore, when the glucose of the culture medium is exhausted, the bacteria metabolize the lactic acid which is present in the medium, leading to a rise in the pH.

Japanese Application JP 04-36180 and Patent EP 430 406, both filed in the name of MEIJI MILK PRODUCTS CO. LTD., propose using these culture conditions to improve the production yield of diacetyl and acetoin by *L. lactis* strain 3022. Application JP 04-36180 also reports an increase in the production of nisin by the strain *L. lactis* K-1, another high diacetyl-producing strain.

The studies above were all performed using diacetyl producing strains, and improved growth properties are strictly associated with greater diacetyl production. As diacetyl and nisin, the products showing high yields in these studies, are both toxic to bacteria, the state and metabolic activity of the bacterial cells at the end of growth cannot be predicted. Thus the above documents do not address the problem of survival of the cultures in the presence of high concentrations of diacetyl (or of nisin), or of the ability of the cultures to start new cultures for classical fermentation.

In addition to stress conditions encountered during growth, bacteria are also subjected to stress when they are harvested, packaged and stored. In particular, oxidative, osmotic or thermal stresses occur at that time.

These various factors alter the survival of the bacteria that constitute the starter culture and can therefore affect their capacity for culture restart when used in fermentations. Indeed, it is notable that differences between batches of starter cultures can be substantial and can lead to products of variable quality.

In order to solve the problems associated with the decrease in pH, the current methods of producing starter cultures of lactic acid bacteria use culture media which are buffered to around pH 6 with cations that are associated with carbonates, hydroxides, phosphates or oxides. However, these additions to the culture medium can cause problems for subsequently made products. For example, adding calcium ions during neutralization can promote the development of phages, and the use of starter cultures which contain phosphate or citrate ions can lead to a lower yield of a cheese product since these molecules increase the solubility of caseins.

In the above documents no information is provided with regard to the possible effects of these culture conditions on the viability of the bacteria and their ability to restart growth if they are once again placed under conventional fermentation conditions.

The present invention solves these problems through a novel process for preparing starter cultures comprised of lactic acid bacteria resulting in an improved yield, and increased survival of said bacteria, and in an improvement of acidifying properties of said starter cultures.

Thus, the inventors observed that the addition of a porphyrin compound to the culture medium of lactic acid bacteria that are cultured under aeration, not only increased the yield of the cultures but also the viability of the bacteria and their resistance to the various stress conditions that can occur during the culture and during the packaging and storage of the starter cultures.

The present invention relates to the use of a porphyrin compound in association with an aerobic culture for increasing the survival of lactic acid bacteria at the end of the said culture. In particular, the present invention relates to a process for preparing a lactic acid bacterial starter culture, for which the process comprises:
- culturing at least one strain of lactic acid bacteria under aeration and in an appropriate nutrient medium in which at least one porphyrin compound is present or is added;
- harvesting the bacteria at the end of the said culture.

"Lactic acid bacteria" refers to a group of bacteria that belong to various genera and that are used in processes for fermenting food products. This group is principally composed of bacteria in which the main product of carbohydrate metabolism is lactic acid. However, bacteria that produce low quantities of lactic acid (*Leuconostoc* and propionic acid bacteria) are included in this list due to their use in fermentation processes. In general, the lactic acid bacteria concerned are those belonging to the genera *Lactococcus, Lactobacillus, Leuconostoc, Propionibacterium,* and *Bifidobacterium,* or *Streptococcus salivarius.*

"Lactic acid bacterial starter culture" refers to any preparation that is intended for inoculating a medium to be fermented and that comprises a bacterial strain or a mixture of strains belonging to one of the above-mentioned genera; such a starter culture can also comprise, or consist of, strains of mutant bacteria and/or strains of recombinant bacteria that are derived from bacteria belonging to the above-mentioned genera.

For the purpose of implementing the present invention, it is possible to use any nutrient medium that is suitable for growing the strain(s) or species of lactic acid bacteria concerned. Such media, which are known per se, usually contain a carbon source, in the form of sugar(s) that can be assimilated, a nitrogen source, generally in the form of a mixture of amino acids, as well as mineral salts and vitamins.

"Porphyrin compound" refers to cyclic tetrapyrrole derivatives whose structures are derived from that of porphyrin by substitution of the carbons located at the apices of the pyrrole core, by various functional groups. It also refers to complexes of the said derivatives with a metal atom that forms coordinate bonds with two of the four nitrogens of the porphyrin ring.

This definition encompasses, but is not limited to:
- uroporphyrins, coproporphyrins, protoporphyrins and haematoporphyrins, as well as their salts and esters and their complexes with a metal atom, preferably an iron atom; the dihydrochloride of coproporphyrin I, the tetraethyl ester of coproporphyrin III, the disodium salt of protoporphyrin IX, the dichloride of haematoporphyrin IX, the tetraisopropyl ester or the tetramethyl ester of coproporphyrin, the tetraisopropyl ester or the tetramethyl ester of coproporphyrin III, haematoporphyrin IX, haemoglobin, protoporphyrin IX, the dimethyl ester of protoporphyrin IX, zinc protoporphyrin IX, hematin and cytohemin;
- the various chlorophylls, in particular chlorophyll a and chlorophyll b, their derivatives such as chlorophyllins, and also their salts and esters, and their complexes with a metal atom, preferably an iron atom.

Particularly preferred porphyrin compounds are protoporphyrin IX and its complexes with an iron atom, in particular haem and hemin, and the derivatives of chlorophyll, such as chlorophyllins.

The inventors observed that positive results with regard to bacterial survival are obtained not only when the porphyrin compound added to the medium contains iron (for example haem), but also when compounds such as protoporphyrin, which does not contain any iron, are added. They have observed that, in the latter case, the porphyrin moiety forms a complex with iron, either present in the media, or by an enzymatic reaction inside the cells, and that the complex thus formed can be incorporated into the bacterial cytochromes.

According to one preferred embodiment of the process of the invention, the said porphyrin compound is added at a concentration of approximately 2.5 to approximately 100, preferably at a concentration of approximately 5 to approximately 40, micromoles per litre of culture medium.

According to another preferred embodiment of the present invention, the culture is aerated so as to maintain, during the entire duration of the culture, an oxygen content which is equal to at least 5 millimoles per litre of culture medium, preferably from 8 to 45 millimoles per litre of culture medium. Aeration can be effected by any means known by one skilled in the Art, for example by shaking or stirring the culture medium, or by passing a gaseous mixture containing oxygen such as air, into the culture medium.

When the present invention is implemented, a bacterial biomass is obtained which is more substantial than that obtained when starter cultures are prepared by conventional methods. Furthermore, there is a greater percentage of viable bacteria that are metabolically active in the bacterial population.

Despite this substantial bacterial growth, the medium is found to be only weakly acidified; the pH decreases less rapidly than in the case of a culture that is not aerated and in which there is no porphyrin compound, and this pH generally stabilizes at a value varying between approximately 5 and approximately 7. When the present invention is implemented, the decrease in the pH is regular; the pH is not found to fall and then rise again, contrary to what KANEKO et al. observed in the case of the *L. lactis* strain 3022.

In the process of the invention, the quantity of glucose in the culture medium converted into lactic acid is less than approximately 40% by weight of the total quantity of glucose initially present. The quantity usually varies between approximately 5% and approximately 30% of the total quantity of glucose that is initially present.

The bacteria are harvested when it is considered that the bacterial population has reached a sufficiently high level. In the methods of the prior art, it is difficult to control the time of harvesting precisely; thus, while it is necessary to have achieved a sufficiently high bacterial population, it is also a requirement that the said bacterial population should still contain a maximum of viable bacteria. On the other hand, use of the process according to the invention makes it possible, at one and the same time, to increase the growth of the bacteria and their viability, thereby providing a much wider latitude for carrying out the harvesting.

For example, it is possible to harvest the bacteria from 5 to 24 hours, advantageously from 7 to 13 hours, after the start of the culture.

Harvesting of bacterial cells to be used for starter culture may be effected by any means known by one skilled in the Art; for example, the culture can be aliquoted into appropriate containers and stored in this form until used; however, bacteria are preferably separated from the culture medium and concentrated by centrifugation or filtration. The harvested bacteria can then be packaged for subsequent use or storage.

The starter cultures that are thus obtained may be used immediately; however, they will most frequently be stored prior to use. The process of the invention has beneficial effects on bacterial viability and metabolic activity, particularly as observed in relation to the properties of the starter cultures after periods of storage.

The starter cultures obtained in accordance with the invention may be stored for some time at the temperature used to grow the bacterial culture (i.e. from approximately 20°C to approximately 50°C, depending on the optimum growth temperature of the lactic acid bacteria concerned); under these conditions, the bacteria still exhibit a substantial survival rate 2 to 4 days after the growth phase of the culture has been terminated; 7 days after the growth phase has been terminated, bacteria exhibit a survival rate that is much greater, compared to cultures treated in the same way, except that growth is carried out without aeration and in the absence of any porphyrin compounds.

For bacterial cultures obtained according to the process of the invention, and then stored at a temperature of approximately 4°C, the survival rate is generally at least 80%, preferably from 90 to 100%, up to at least 7 days after the growth phase has been terminated; it is still possible to observe a survival rate of between 0.1 to 10% 2 months after the growth phase has been terminated.

In order to obtain an even longer storage period, as well as to facilitate transport and storage, it is possible to freeze or lyophilize the starter cultures or else pulverise or atomize them in vacuo. These various treatments, as well as the storage of the starter cultures once they have been treated, are effected using techniques that are known to one skilled in the Art.

In the case of freezing, it can be advantageous to use a cryoprotectant, selected from among the following compounds:
glucose, lactose, raffinose, sucrose, trehalose; adonitol, glycerol, mannitol, methanol, polyethylene glycol, propylene glycol, ribitol; alginate, bovine serum albumin, carnitine, citrate, cystein, dextran, dimethyl sulphoxide, sodium glutamate, glycine betaine, glycogen, hypotaurine, skimmed milk, peptone, polyvinyl pyrrolidine and taurine.

The cryoprotectant employed is advantageously alginate, glycerol, glycine betaine, skimmed milk, trehalose or sucrose.

The present invention also encompasses the lactic acid bacterial starter cultures that can be obtained by the process according to the invention. These starter cultures can comprise one or more species of lactic acid bacteria and/or one or more strains of one and the same species, with all or some of the said species or the said strains having been cultured in accordance with the invention. Several different species or several different strains may be cultured simultaneously (when their optimal growth conditions are compatible) or else cultured separately and combined after harvesting.

The lactic acid bacterial starter cultures which have been obtained in accordance with the invention can be used to inoculate a medium to be fermented, in particular within the context of transforming raw materials of animal or plant origin, for example for producing food products, such as fermented dairy products, or for producing molecules of interest in a fermenter.

Upon inoculation of a starter culture prepared according to this invention, and using any of a variety of lactococcal strains and subspecies, one observes that the culture starts growth, and acidifies rapidly, even after the starter culture has been stored for a long period, which demonstrates that a substantial proportion of bacteria in the starter culture are viable and metabolically active, and furthermore that these bacteria, which are derived from cultures carried out in the presence of a porphyrin compounds and under aeration, are able to readapt rapidly to the usual conditions of lactic acid fermentation.

The invention will be illustrated in more detail in the description that follows and that refers to non-limiting examples of the implementation of the process of the invention.

### I) Figure legends

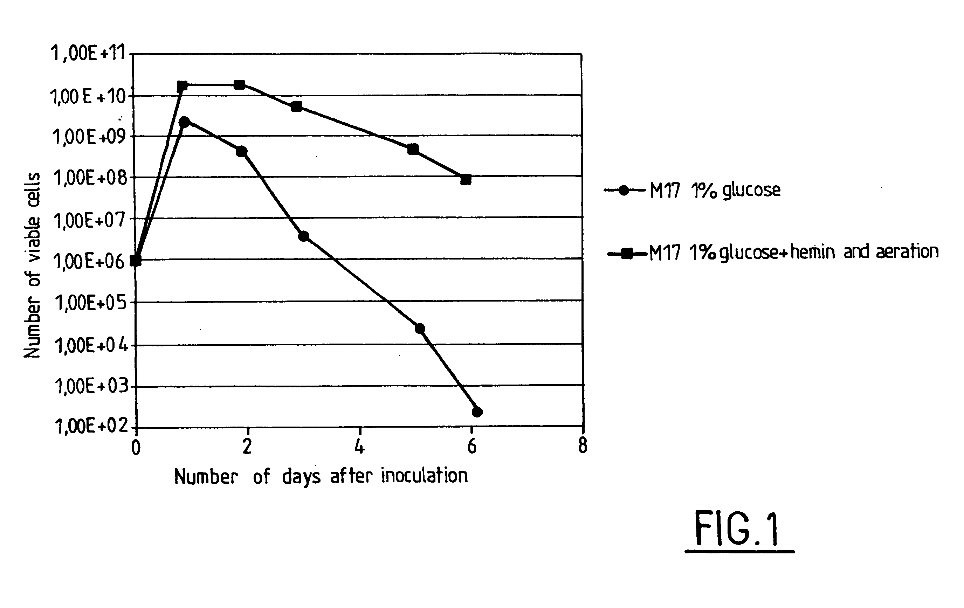

- Figure 1: Growth and survival of *Lactococcus lactis,* which is cultured and stored at 30°C.
   This figure compares the growth and survival curves of lactic acid bacteria (in this case *Lactococcus lactis*) that are cultured at 30°C with or without hemin and aeration according to the process of the invention. The number of viable cells is expressed as a function of time (number of days after inoculation, which is time zero), with the storage temperature of the bacteria being 30°C. The curve with the circles (●) corresponds to bacteria cultivated by the method of the prior art (without hemin), whereas that with the squares (■) corresponds to the culture conditions as described in this invention, in the presence of hemin and with aeration of the medium.

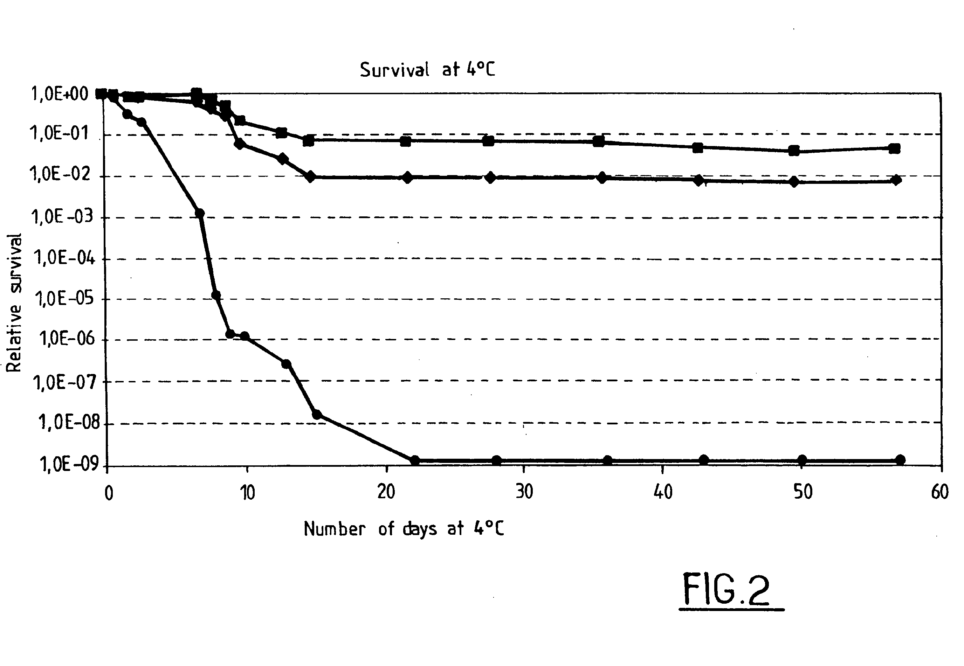

- Figure 2: Growth and survival of *Lactococcus lactis* cultured at 30°C for 24 h and then stored at 4°C.
   This figure compares the growth and survival curves when the lactic acid bacteria (in this case *Lactococcus lactis*) are cultured at 30°C and transferred to 4°C, 24 h after inoculation. The culture phase at 30°C is carried out in a medium without hemin or aeration (●), or else under aeration and either in the presence of hemin (■) or of protoporphyrin IX (◆). The number of viable cells is expressed as a function of time (number of days after inoculation).

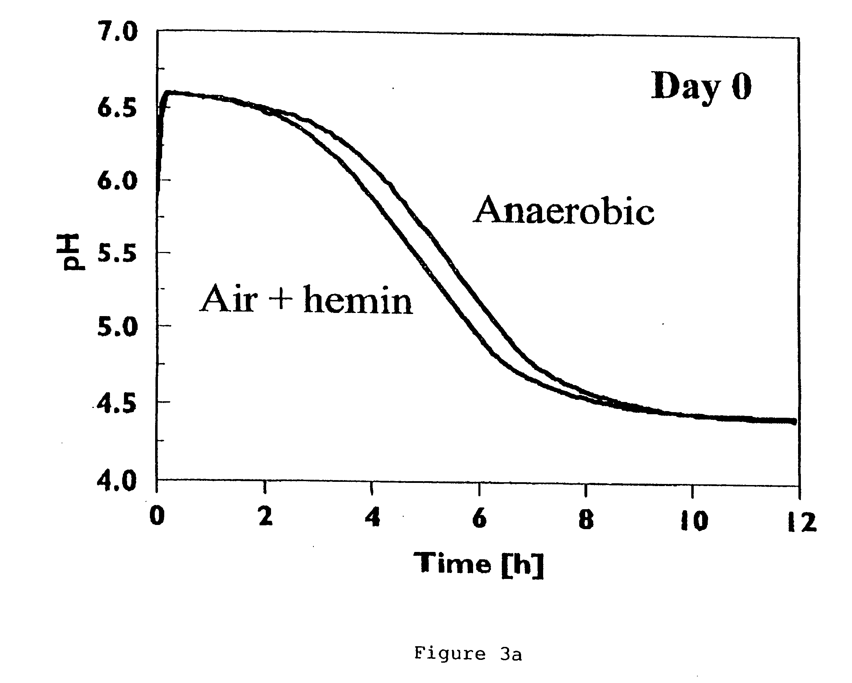

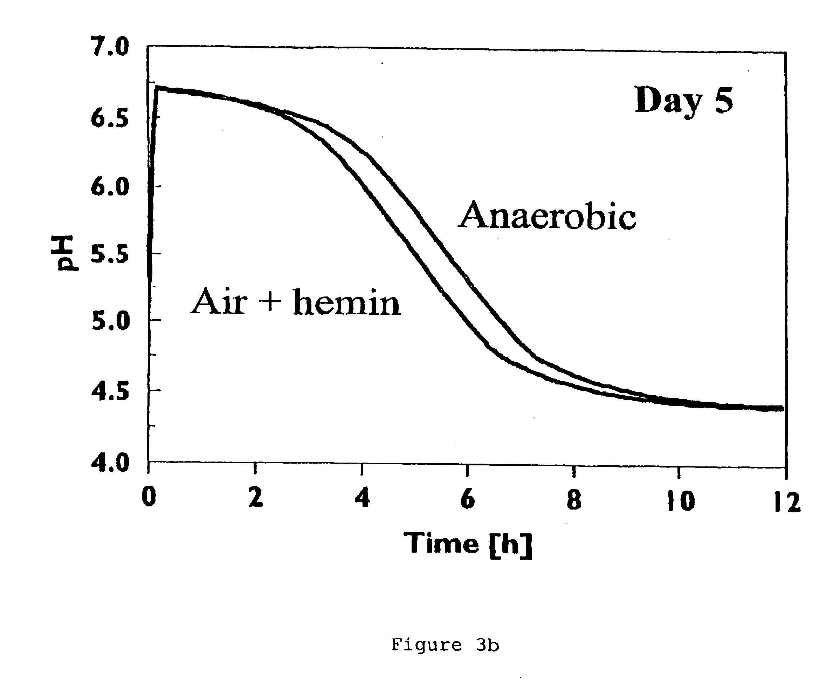

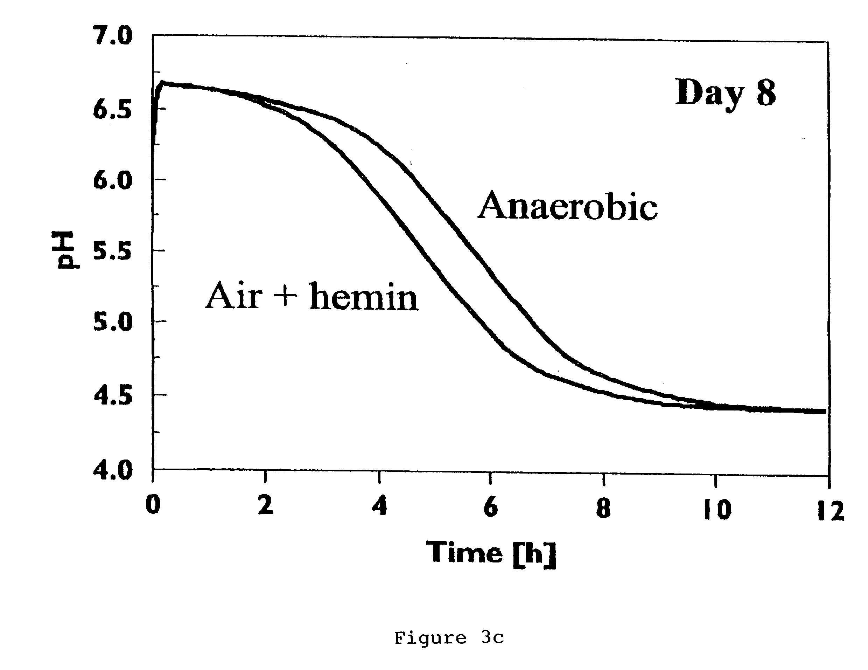

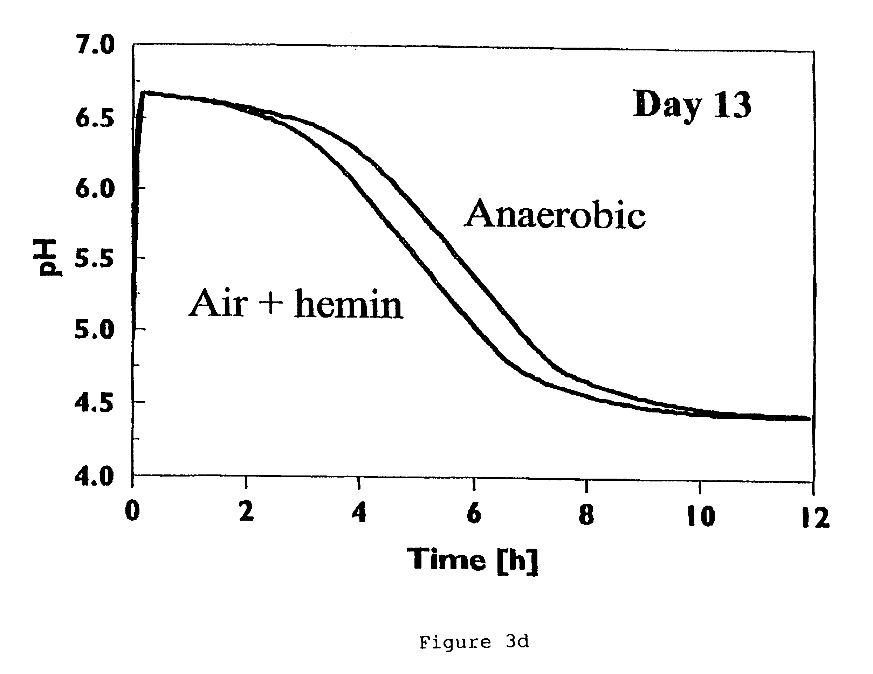

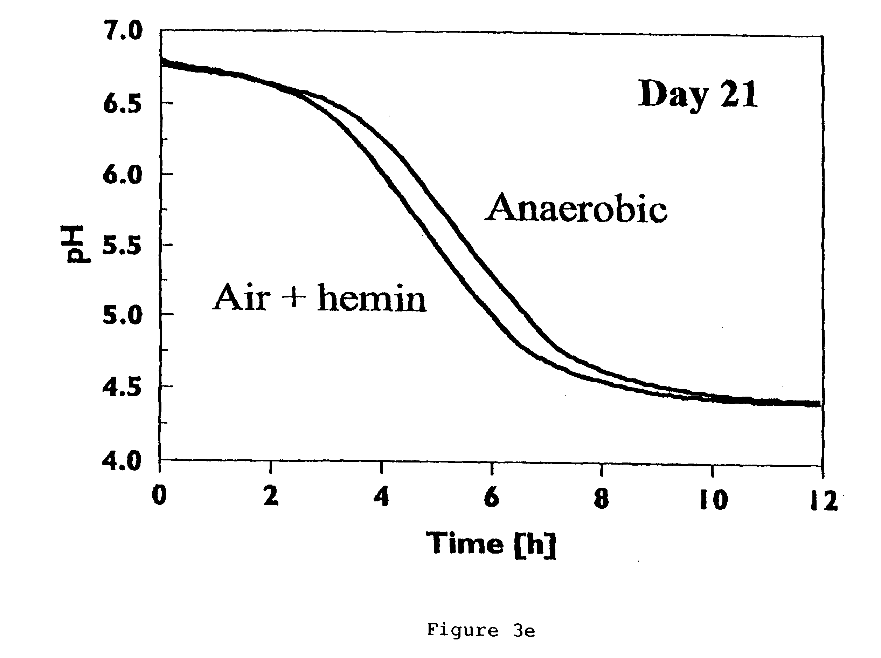

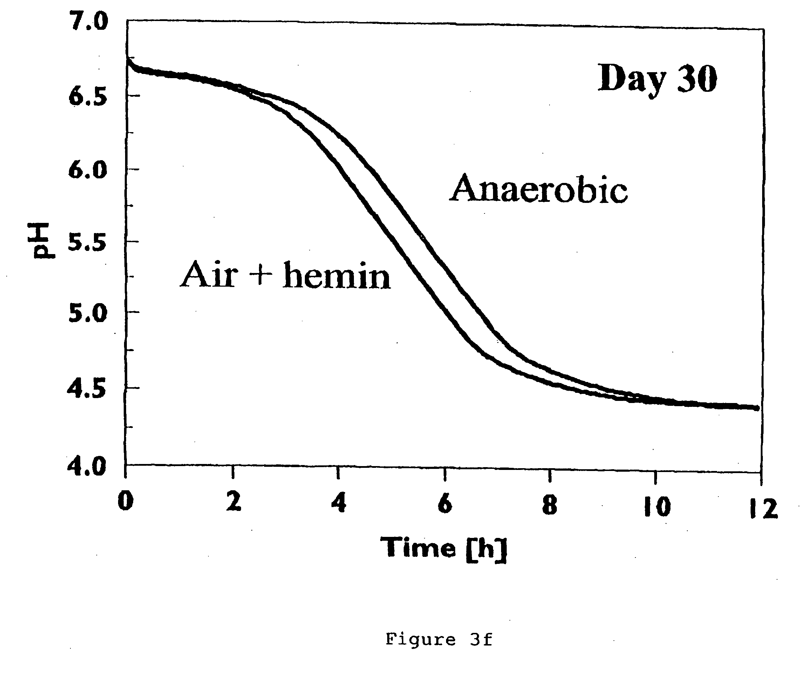

- Figure 3 (a-f): Acidification properties of a lactic acid bacterial starter culture prepared either according to the invention, or traditionally under non-aerobic conditions. The strain used, CHCC373, is a *Lactococcus lactis* starter culture strain.
   These figures plot the variation in pH as a function of time during fermentation of a dairy substrate by a starter culture that was prepared in accordance with the invention (air+hemin), or by a starter culture that was prepared by culturing under non-aerobic conditions (anaerobic), immediately after the culture (a), or after 5 days (b), 8 days (c), 13 days (d), 21 days (e) and 30 days (f) of storage at 4°C.

### II) Examples

*Lactococcus lactis* is a bacterium that, when cultured in a rich medium, in milk or in accordance with the art prior to this invention, exhibits the following characteristics:
- production of lactic acid as the main product of the metabolism of sugars (glucose or lactose, for example),
- acidification of the medium to a pH value of approximately 4.5, in a medium containing 1% glucose.

However, when the bacteria are cultured under the conditions described in the present invention, it can be observed that these characteristics are modified. The following experiments demonstrate this.

### EXAMPLE 1: PROPERTIES OF BACTERIAL CULTURES GROWN IN THE PRESENCE OF OXYGEN AND A PORPHYRIN DERIVATIVE; EFFECTS ON BACTERIAL YIELD AND ACIDIFICATION OF THE MEDIUM.

### 1) Culturing in the presence of hemin

A laboratory medium (M17 supplemented with glucose in amounts as indicated in the Table I, lactose being also usable) is inoculated with the *Lactococcus lactis* subsp. *cremoris* strain MG1363 in the form of a 1/100 or 1/1000 dilution of a saturated culture (prepared at 30°C with no shaking). The inoculated medium contained or not hemin at a final concentration of 10 µg/ml (a 0.5 mg/ml stock solution is prepared by dissolving 100 mg of hemin in 2 ml of 5N NaOH, to which 198 ml of water is then added; the solution is autoclaved at 120°C for 20 minutes). For bacteria grown in medium containing hemin, cultures were maintained at 30°C with shaking (250 rotations per minute) in order to ensure oxygenation. Control cultures, without addition of hemin and without shaking, were grown at 30°C in parallel. After 24 h of growth, aliquots are removed to measure the optical density at 600 nm (OD_{600 nm}), the number of viable bacteria, the final pH and the concentration of lactic acid in the medium. The results are shown in Table I.

**Table I**

| A : Cultures without hemin or aeration | | | | | | | | | | | | |
|---|---|---|---|---|---|---|---|---|---|---|---|---|
| Concentration of glucose (g/l) | 5 | | 6 | | 7 | | 8 | | 9 | | 10 | |
| Dilution factor | 100 | 1000 | 100 | 1000 | 100 | 1000 | 100 | 1000 | 100 | 1000 | 100 | 1000 |
| OD_{600 nm} | 2.8 | 2.4 | 2.22 | 2.37 | 2.4 | 2.78 | 2.82 | 2.8 | 3.05 | 2.95 | 3.1 | 2.8 |
| Viable bacterial count (×10⁹) | 3.25 | 3.44 | 3.43 | 3.6 | 3.2 | 3.2 | 3.5 | 3.5 | 3.5 | 3.7 | 3.9 | 2.4 |
| pH | 5.77 | 5.74 | 5.51 | 5.52 | 5.13 | 5.19 | 4.96 | 4.92 | 4.71 | 4.65 | 4.56 | 4.55 |
| Concentration of lactic acid (g/l) | not measured | 3.3 | not measured | 4.8 | not measured | 4.9 | not measured | 6.5 | not measured | 6.9 | not measured | 8 |
| | | | | | | | | | | | | |

| B : Cultures with hemin and aeration | | | | | | | | | | | | |
|---|---|---|---|---|---|---|---|---|---|---|---|---|
| Concentration of glucose (g/l) | 5 | | 6 | | 7 | | 8 | | 9 | | 10 | |
| Dilution factor | 100 | 1000 | 100 | 1000 | 100 | 1000 | 100 | 1000 | 100 | 1000 | 100 | 1000 |
| OD_{600 nm} | 4.6 | 4.5 | 5.1 | 5.5 | 5.6 | 5.7 | 5.9 | 5.4 | 5.9 | 5.8 | 6.1 | 6.2 |
| Viable bacterial count (×10⁹) | 5.7 | 5.7 | 6 | 7.6 | 9.1 | 8.6 | 8.9 | 9.3 | 9.2 | 9.4 | 10.1 | 9.6 |
| pH | 6.2 | 6.19 | 6.25 | 6.13 | 6.09 | 6.07 | 6.04 | 6.03 | 6.02 | 6.01 | 6.01 | 5.99 |
| Concentration of lactic acid (g/l) | not measured | 0.12 | not measured | 0.14 | not measured | 0.19 | not measured | 0.35 | not measured | 0.52 | not measured | 0.98 |

These results show that a greater biomass is achieved when the bacteria are cultured in the presence of hemin and oxygen. This increase is demonstrated by the higher optical density values. Furthermore, a higher number of viable cells is observed when the cells are cultured in the presence of hemin and oxygen. It may also be noted that, when the cells are cultured in the presence of hemin and oxygen, the pH does not vary greatly and remains stable around a value of approximately 6.1 whatever the concentration of glucose. In contrast, in the case of cells cultured under conventional conditions, the pH is markedly lower at a glucose concentration of 1% (pH 4.5) than when the glucose concentration is at 0,5% (pH 5.7). It is also observed that the production of lactic acid by cells cultured in the presence of hemin and oxygen is low and is always less than 10% of the quantity of sugar added, whereas, in the case of the control cultures, approximately 80% of the added glucose is converted to lactic acid.

### 2) Culturing in the presence of protoporphyrin IX

A laboratory medium (M17 supplemented with 1% glucose) is inoculated with the *Lactococcus lactis* subsp. *cremoris* strain MG1363 in the form of a 1/1000 dilution of a saturated culture (prepared at 30°C with no shaking). The inoculated medium contained or not protoporphyrin IX at a final concentration of 10 µg/ml (a 0,5 mg/ml stock solution is prepared by adding 100 mg of protoporphyin IX to 2 ml of 5N NaOH, to which 198 ml of water is then added; the solution is autoclaved at 120°C for 20 minutes). For bacteria grown in medium containing protoporphyrin IX, cultures were maintained at 30°C with shaking (250 rotations per minute) in order to ensure oxygenation, or without shaking as a control. Two other control cultures, without protoporphyin IX or without shaking, were grown at 30°C in parallel. After 24 h of growth, aliquots are removed to measure the optical density at 600 nm (OD₆₀₀ₙₘ), the number of viable bacteria, the final pH and the concentration of lactate in the medium. The results are compiled in Table II.

**Table II**

| Culturing conditions | -P-O₂^{a} | -P+O₂^{b} | +P-O₂^{c} | +P+O₂^{d} |
|---|---|---|---|---|
| OD_{600 nm} | 2 | 2.6 | 2.5 | 4.6 |
| Number of viable cells (×10⁹) | 2.8 | 2.9 | 2.7 | 6.9 |
| Final pH | 4.52 | 4.61 | 4.53 | 5.49 |
| Concentration of lactic acid (g/l) | 8.9 | 8.2 | 8.2 | 1.9 |

| | | | | |
|---|---|---|---|---|
| a: without protoporphyrin IX or aeration b: without protoporphyrin IX but with aeration c: with protoporphyrin IX but without aeration d: with protoporphyrin IX and aeration | | | | |

The OD₆₀₀ values show that a greater biomass is achieved when the cells are cultured in the presence of protoporphyrin IX and oxygen. Furthermore, the number of viable cells is higher. It may also be noted that when the cells are cultured in the presence of protoporphyrin IX and oxygen, the pH does not vary greatly and remains stable at a value of approximately 5.5. In contrast, the pH decreases strongly (final pH of 4.5) in the case of the control cultures. It is also observed that the production of lactic acid by the cells cultured in the presence of protoporphyrin IX and oxygen is low and is always less than 40% of the quantity of sugar added, whereas, in the case of the control cultures, 80% of the added glucose is converted to lactic acid.

The experiments described in 1) and 2) above demonstrate that, in cultures carried out in accordance with the invention, the bacterial mass (measured by turbidimetry) is approximately 2 times higher and the population of viable bacteria is 2 to 5 times higher, than in the case of a culture carried out without aeration and in the absence of a porphyrin compound.

### 3) Culturing in the presence of chlorophyllin

A laboratory medium (M17 supplemented with 1% glucose) is inoculated with the *Lactococcus lactis* subsp. *cremoris* strain MG1363 in the form of a 1/1000 dilution of a saturated culture (prepared at 30°C with no shaking). The inoculated medium contained or not chlorophyllin (a degradation product of chlorophyll) at a final concentration of 10 µg/ml. In order for it to be active, the chlorophyllin was incubated beforehand in an acidic medium and in the presence of traces of iron in order to replace the Cu atom with an Fe atom. This can, for example, be done as follows:
a) by incubating a concentrated solution of chlorophyllin for 24 h in a laboratory medium (M17 supplemented with glucose) which was inoculated with 10⁶ lactococci per ml and maintained at 30°C for 24 h. The supernatant containing the chlorophyllin at a concentration of 100 µg/ml is filtered and then added to the culture medium so as to obtain a final concentration of 10 µg/ml.
b) by incubating a concentrated solution of chlorophyllin for 24 h in M17 at a pH of between 3 and 5 (acidified with HCl) and at a temperature of 4°C, 30°C or 60°C. The pH is then readjusted to 7. The chlorophyllin solution, at a concentration of 100 µg/ml, is subsequently filtered and then added to the culture medium in order to obtain a final concentration of 10 µg/ml.
c) by incubating a concentrated solution of chlorophyllin for 24 h in M17 at a pH of 4.5 (acidification by adding lactate) and at a temperature of 4°C, 30°C or 60°C. The pH is then readjusted to 7. The chlorophyllin solution, at a concentration of 100 µg/ml, is subsequently filtered and then added to the culture medium in order to obtain a final concentration of 10 µg/ml.

For bacteria grown in medium containing chlorophyllin, cultures are placed at 30°C, with shaking in order to oxygenate the cultures (250 rotations per minute). Control cultures, to which no chlorophyllin is added and which are not shaken, are grown in parallel. After 24 h of growth, aliquots are removed for measuring the optical density at 600 nm (OD₆₀₀ₙₘ) and the final pH.

In all the cultures carried out using preparations a), b) or c), and under aeration, an OD_{600 nm} which is higher than that of the control cultures by from 0.3 to 0.8 units, and a pH which is higher than that of the control cultures by from 0.3 to 0.8 units, are observed, depending on the samples concerned. No significant variation from the control is observed when the cultures carried out in the presence of chlorophyllin are not shaken.

### EXAMPLE 2: EFFECT OF CULTURING IN THE PRESENCE OF OXYGEN AND OF A PORPHYRIN DERIVATIVE, ON THE SURVIVAL OF THE LACTIC ACID BACTERIA DURING STORAGE AT 30°C

A laboratory medium, i.e. M17 supplemented with 1% of glucose, is inoculated with *Lactococcus lactis* subsp. *cremoris* strain MG1363 in the form of a 1/1000 dilution of a saturated culture. The cultures are then divided into two equal parts, and hemin is added to one of these two cultures to give a final concentration of 10 µg/ml. The control culture, which does not contain any hemin, is incubated at 30°C without shaking, whereas that containing hemin is incubated at 30°C with shaking (250 rotations per minute) in order to oxygenate it. Aliquots of the two cultures are removed regularly during the exponential growth phase in order to monitor viability, rate of growth and pH. After 24 h of growth, the cultures are placed (stored) in a 30°C incubator without shaking, and aliquots are removed every 24 h in order to monitor the viability of the cells. The results are shown in Table III and Figure 1.

**Table III**

| Number of days after inoculation | 0 | 1 | 2 | 3 | 5 | 6 |
|---|---|---|---|---|---|---|
| 1% glucose M17 | 9.5×10⁵ | 2.45×10⁹ | 4.5×10⁸ | 3.8×10⁶ | 2.2×10⁴ | 2.2×10² |
| 1% glucose M17 + hemin and aeration | 1×10⁶ | 1.76×10¹⁰ | 1.71×10¹⁰ | 5.32×10⁹ | 4.3×10⁸ | 8.5×10⁷ |

The cells cultured in the presence of hemin and oxygen survive to a much greater extent than do the cells cultured under the conventional conditions. This improvement in survival is evident after one day of storage (that is to say, 2 days after inoculation); the cells that are cultured in the presence of hemin and oxygen do not lose any viability. In contrast, the number of viable cells in the control cultures has already decreased markedly. This difference increases during storage: after 5 days of storage at 30°C, the rate of survival is approximately 10⁻⁷ in the case of the control cultures whereas it is approximately 10⁻² in the case of the cultures that were grown with aeration and in the presence of hemin.

### EXAMPLE 3: EFFECT OF CULTURING IN THE PRESENCE OF OXYGEN AND A PORPHYRIN DERIVATIVE, ON THE SURVIVAL OF THE LACTIC ACID BACTERIA DURING STORAGE AT 4°C

The *Lactococcus lactis* subsp. *cremoris* strain MG1363 was cultured as in the experiment described in Example 2, but the storage, which is carried out 24 h after inoculation, is at 4°C instead of at 30°C. The results are shown in Table IV and Figure 2.

These results demonstrate that the cells cultured in the presence of hemin or protoporphyrin IX and oxygen survive to a significantly greater extent during storage in the cold (4°C) than do cells cultured under conventional conditions. After 2 months at 4°C, the bacteria that were cultured in the presence of hemin and under aeration exhibit a survival rate of the order of 5%, and the bacteria that were cultured in the presence of protoporphyrin IX and under aeration, exhibit a survival rate of the order of 1%. In contrast, survival of control cells is less than 10⁻⁹

**Table IV**

| | Number of cells per ml | | | | | | | |
|---|---|---|---|---|---|---|---|---|
| Number of days after culture | 0 | 1 | 2 | 3 | 7 | 8 | 9 | 10 |
| 1% glucose M17 + hemin and aeration | 4.3×10⁹ | 4.2×10⁹ | 3.8×10⁹ | 3.8×10⁹ | 4.2×10⁹ | 2.8×10⁹ | 2.2×10⁹ | 9.6×10⁸ |
| 1% glucose M17 + protoporphyrin IX and aeration | 6.4×10⁹ | 6.2×10⁹ | 5.6×10⁹ | 5.4×10⁹ | 4.0×10⁹ | 2.8×10⁹ | 1.9×10⁹ | 3.9×10⁸ |
| 1% glucose M17 | 1.6×10⁹ | 1.3×10⁹ | 5.3×10⁸ | 3.5×10⁸ | 2.0×10⁶ | 2.0×10⁴ | 2.1×10³ | 1.9×10³ |
| | | | | | | | | |

| | Number of cells per ml | | | | | | | |
|---|---|---|---|---|---|---|---|---|
| Number of days after culture | 13 | 15 | 22 | 28 | 36 | 43 | 50 | 57 |
| 1% glucose M17 + hemin and aeration | 5.0×10⁸ | 3.2×10⁸ | 3.0×10⁸ | 3.0×10⁸ | 2.9×10⁸ | 2.2×10⁸ | 1.8×10⁸ | 2.0×10⁸ |
| 1% glucose M17 + protoporphyrin IX and aeration | 1.7×10⁸ | 6.0×10⁷ | 5.9×10⁷ | 5.4×10⁷ | 5.4×10⁷ | 5.3×10⁷ | 4.8×10⁷ | 5.3×10⁷ |
| 1% glucose M17 | 4.0×10² | 2.4×10¹ | <2.0×10⁰ | <2.0×10⁰ | <2.0×10⁰ | <2.0×10⁰ | <2.0×10⁰ | <2.0×10⁰ |

### EXAMPLE 4 : EFFECTS OF THE CULTURE PROCESS OF THE INVENTION ON THE YIELD, AND MEDIUM ACIDIFICATION PROPERTIES, USING DIFFERENT STRAINS OF LACTIC ACID BACTERIA

Bacteria [MG1363 and 7 strains (IL1403, IL582, IL801, IL896, Z105, Z106, Z191) representative of *L. lactis* subgroups [TAILLEZ et al., System. Appl. Microbiol., 21, 530-538, (1998)] are cultured in M17 medium (1% glucose) inoculated (1/1000 dilution) with a saturated starter culture previously grown at 30°C for 24 h. Hemin at a final concentration of 10 µg/ml is added to the inoculated medium before aerating the cultures by shaking (minimum 220 rpm). Control cultures are grown in parallel with no hemin in the medium, and no shaking. After 24 h., aliquots are taken and OD₆₀₀ and final pH are measured. The data are shown in Table V below.

**Table V**

| Culture with no hemin and no shaking | MG1363 | IL1403 | IL582 | IL801 | IL896 | Z105 | Z106 | Z191 |
|---|---|---|---|---|---|---|---|---|
| OD₆₀₀ₙₘ | 2,4 | 1,7 | 2,1 | 2,1 | 2,5 | 2,2 | 2,3 | 2,3 |
| pH | 4,59 | 4,57 | 4,57 | 4,53 | 4,55 | 4,59 | 4,58 | 4,59 |
| Culture with hemin and agitation | MG1363 | IL1403 | IL582 | IL801 | IL896 | Z105 | Z106 | Z191 |
| OD₆₀₀ₙₘ | 5 | 5,3 | 5,1 | 4,3 | 4,5 | 5 | 4,5 | 3,8 |
| pH | 5,86 | 5,41 | 5,73 | 5,44 | 5,24 | 5.67 | 5,74 | 5,36 |

These data show that the effect observed with MG1363 is also obtained with *Lactococci* of various subgroups. Note that all the bacteria cultivated according to the invention acidify less (final pH is always higher than 5.2 compared to a final pH of 4.5 for the control cultures), and that the biomass is always greater in the cultures obtained according to the invention (OD higher than 4.3 compared to an OD lower than 2.5 for the control).

### EXAMPLE 5: PROPERTIES OF A STARTER CULTURE WHICH HAS BEEN PREPARED IN ACCORDANCE WITH THE INVENTION

An industrial strain of *L. lactis* subsp. *lactis* (strain CHCC373 available from CHR HANSEN or from the Deutsche Sammlung von Microorganismen und Zellkulturen, Mascheroder Weg 1b, D-38124 BRALTNSCHWEIG, deposited in February 1998, under the accession number DSM12015) is cultured at 30°C in a 350 litre fermenter, either a) under anaerobic conditions under an atmosphere of nitrogen, or b) in the presence of 20 ppm of hemin and with a flow of air. When growth is terminated, the cultures are centrifuged and the pellets are frozen in liquid nitrogen and then stored at -80°C.

The acidification properties of the starter cultures prepared by conventional means a) or by the process of the invention b) are measured at day 0 and after 5, 8, 13, 21 and 30 days at -80°C. The growth medium, 9.5% reconstituted skimmed milk, is inoculated with 0.01% (w/v) of starter culture 'a)' or 'b)', as above, and the fermentation is carried out at 30°C without shaking; the pH is measured continuously.

The results are shown in Figure 3 (a-f). These results demonstrate that:
- neither of the 2 starter cultures loses its properties during storage at -80°C;
- from the time of its preparation, and during the entire storage period, starter culture b) according to the invention exhibits properties significantly superior to those of starter culture a), which is obtained using conventional methods. In particular, starter culture b) reaches a predetermined pH 30 to 40 minutes before starter culture a), while showing the same acidification kinetics.

It appears therefore, that the addition of hemin combined with culturing under aerobic conditions significantly improves the production of a lactic acid bacterial starter culture, and in particular the acidification performance of the said starter culture.

## Claims

1. Process for preparing a lactic acid bacterial starter culture, which comprises:
- culturing at least one strain of lactic acid bacteria under aeration and in an appropriate nutrient medium, in which at least one porphyrin compound is present or is added;
- harvesting the bacteria at the end of the said culture.

2. Process according to Claim 1, in which the lactic acid bacteria are selected from *Lactococcus, Lactobacillus, Leuconostoc, Propionibacterium, Bifidobacterium,* and *Streptococcus salivarius.*

3. Process according to either Claim 1 or 2, **characterized in that** the said porphyrin compound is selected from uroporphyrins, coproporphyrins, protoporphyrins, haematoporphyrins, chlorophylls and chlorophyllin, their salts and esters and their complexes with an iron atom.

4. Process according to any one of Claims 1 to 3, in which the said compound is added at a concentration of approximately 2.5 to approximately 100 micromoles per litre of culture medium.

5. Process according to any one of Claims 1 to 4, in which the culture is aerated so as to maintain, during the whole duration of the culture, an oxygen content which is equal to at least 5 millimoles per litre of culture medium.

6. Process according to any one of Claims 1 to 5, **characterized in that** the bacteria are harvested between 5 and 24 hours after the start of the culture.

7. Process according to any one of Claims 1 to 6, which further comprises packaging the harvested bacteria.

8. Process according to any one of Claims 1 to 7, **characterized in that** it further comprises storage of the harvested lactic acid bacteria.

9. Process according to Claim 8, **characterized in that** the said lactic acid bacteria are stored at approximately 4°C.

10. Process according to Claim 8, **characterized in that** the said lactic acid bacteria are stored in frozen or lyophilized form.

11. Lactic acid bacterial starter culture obtainable by a process according to any one of Claims 1 to 10.

12. Process for preparing a fermented product, **characterized in that** it comprises seeding a medium to be fermented with a lactic acid bacterial starter culture according to Claim 11.

13. Use of a porphyrin compound combined with culturing under aerobic conditions for increasing the survival of lactic acid bacteria at the end of the said culture.

14. Use of a lactic acid bacterial starter culture according to Claim 11 for preparing a fermented product.

## Patentansprüche

1. Verfahren zur Herstellung einer Milchäurebakterien-Starterkultur, das umfasst :
- Kultivieren wenigstens eines Milchäurebakterien-Stammes unter Belüftung und in einem geeigneten Nährmedium, in dem wenigstens eine Porphyrinverbindung vorhanden ist oder zugesetzt ist ;
- Ernten der Bakterien am Ende der Kultur.

2. Verfahren nach Anspruch 1, bei dem die Milchäurebakterien ausgewählt sind aus *Lactococcus, Lactobacillus, Leuconostoc, Propionibacterium, Bifidobacterium* und *Streptococcus salivarius.*

3. Verfahren nach Anspruch 1 oder 2, **dadurch gekennzeichnet, dass** die Porphyrinverbindung ausgewählt ist aus Uroporphyrinen, Coproporphyrinen, Hämatoporphyrinen, Chlorophyllen und Chlorophyllin, ihren Salzen und Estern und ihren Komplexen mit einem Eisenatom.

4. Verfahren nach einem der Ansprüche 1 bis 3, bei dem die Verbindung zu einer Konzentration von näherungsweise 2,5 bis näherungsweise 100 Mikromol pro Liter Kulturmedium zugesetzt wird.

5. Verfahren nach einem der Ansprüche 1 bis 4, bei dem das Kulturmedium belüftet wird, um während der gesamtem Kulturdauer einen Sauerstoffgehalt aufrecht zu erhalten, der wenigstens 5 Millimol pro Liter Kulturmedium entspricht.

6. Verfahren nach einem der Ansprüche 1 bis 5, **dadurch gekennzeichnet, dass** die Bakterien zwischen 5 und 24 Stunden nach dem Beginn der Kultur geerntet werden.

7. Verfahren nach einem der Ansprüche 1 bis 6, das weiterhin Verpacken der geernteten Bakterien umfasst.

8. Verfahren nach einem der Ansprüche 1 bis 7, **dadurch gekennzeichnet, dass** es weiterhin eine Lagerung der geernteten Milchäurebakterien umfasst.

9. Verfahren nach Anspruch 8, **dadurch gekennzeichnet, dass** die Milchäurebakterien bei näherungsweise 4°C gelagert werden.

10. Verfahren nach Anspruch 8, **dadurch gekennzeichnet, dass** die Milchäurebakterien in gefrorener oder lyophilisierter Form gelagert werden.

11. Milchäurebakterien-Starterkultur, erhältlich durch ein Verfahren nach einem der Ansprüche 1 bis 10.

12. Verfahren zur Herstellung eines fermentierten Produkts, **dadurch gekennzeichnet, dass** es das Beimpfen eines Mediums, das fermentiert werden soll, mit einer Milchäurebakterien-Starterkultur nach Anspruch 11 umfasst.

13. Verwendung einer Porphyrinverbindung kombiniert mit Kultuvieren unter aeroben Bedingungen zur Erhöhung des Überlebens von Milchäurebakterien am Ende der Kultur.

14. Verwendung einer Milchäurebakterien-Starterkultur nach Anspruch 11 zur Herstellung eines fermentierten Produkts.

## Revendications

1. Procédé de préparation de culture starter de bactéries lactiques, comprenant:
- la culture d'au moins une souche de bactéries lactiques en conditions aérées et dans un milieu de nutriments approprié, dans lequel au moins un composant porphyrine est présent ou est ajouté ;
- la collecte des bactéries à la fin de ladite culture.

2. Procédé selon la revendication 1, dans lequel les bactéries lactiques sont sélectionnées entre le *Lactococcus,* le *Lactobacillus,* le *Leuconostoc,* le *Propionibacterium,* le *Bifidobacterium,* et le *Streptococcus salivarius.*

3. Procédé selon la revendication 1 ou 2, **caractérisé en ce que** ledit composé porphyrine est sélectionné parmi les uroporphyrines, les coproporphyrines, les protoporphyrines, les hématoporphyrines, les chlorophylles et chlorophyllines, leurs sels et esters et leurs complexes avec un atome de fer.

4. Procédé selon l'une quelconque des revendications 1 à 3, dans lequel ledit composé est ajouté à une concentration d'environ 2,5 à environ 100 micromoles par litre de milieu de culture.

5. Procédé selon l'une quelconque des revendications 1 à 4, dans lequel la culture est aérée de manière à maintenir, pendant toute la durée de la culture, une teneur en oxygène égale à au moins 5 millimoles par litre de milieu de culture.

6. Procédé selon l'une quelconque des revendications 1 à 5, **caractérisé en ce que** les bactéries sont collectées entre 5 et 24 heures après le début de la culture.

7. Procédé selon l'une quelconque des revendications 1 à 6, comprenant en outre l'étape consistant à conditionner les bactéries collectées.

8. Procédé selon l'une quelconque des revendications 1 à 7, **caractérisé en ce qu'**il comprend en outre le stockage des bactéries lactiques collectées.

9. Procédé selon la revendication 8, **caractérisé en ce que** lesdites bactéries lactiques sont stockées à 4°C environ.

10. Procédé selon la revendication 8, **caractérisé en ce que** lesdites bactéries lactiques sont stockées sous une forme surgelée ou lyophilisée.

11. Culture starter de bactéries lactiques susceptible d'être obtenue par un procédé selon l'une quelconque des revendications 1 à 10.

12. Procédé de préparation d'un produit fermenté, **caractérisé en ce qu'**il comprend une étape d'ensemencement d'un milieu pour qu'il fermente avec une culture starter de bactéries lactiques selon la revendication 11.

13. Utilisation d'un composé porphyrine en association avec une mise en culture dans des conditions d'aérobie pour augmenter la survie des bactéries lactiques acides à la fin de ladite culture.

14. Utilisation d'une culture starter de bactéries lactiques selon la revendication 11 pour préparer un produit fermenté.
